# EUROPEAN PATENT APPLICATION

(11) **EP 4 792 812 A1**
(43) Date of publication of application: **19.08.2026**
(21) Application number: 24877288.1
(22) Date of filing: 11.10.2024
(51) Int. Cl.: A61K 31/444, A61K 9/70, A61K 47/10, A61K 47/12, A61P 25/16, A61P 25/28, A61P 43/00

(54) **PERCUTANEOUS ABSORPTION COMPOSITION**

(30) Priority: 12.10.2023 JP 2023176933
(71) Applicant: MEDRx Co., Ltd., Higashikagawa-shi Kagawa 769-2712 (JP); Alto Neuroscience, Inc., Mountain View, California 94041 (US)
(72) Inventor: NAKAMURA, Jun, Higashikawa-shi, Kagawa 769-2712 (JP); HAMAMOTO, Hidetoshi, Higashikawa-shi, Kagawa 769-2712 (JP); ETKIN, Amit, Mountain View, California 94041 (US); SAVITZ, Adam, Mountain View, California 94041 (US); MORIMOTO, Bruce, Mountain View, California 94041 (US)
(74) Representative: Vossius & Partner Patentanwälte Rechtsanwälte mbB
(86) International application number: PCT/JP2024/036472
(87) International publication number: WO 2025/079694

(57) **Abstract**

The present invention provides a transdermal absorption composition comprising 4-cyclopropylmethoxy-N-(3,5-dichloro-1-oxido-4-pyridin-4-yl)-5-(methoxy)pyridine-2-carboxamide and levulinic acid, wherein the content of levulinic acid is 2 to 6 times the weight of 4-cyclopropylmethoxy-N-(3,5-dichloro-1-oxido-4-pyridin-4-yl)-5-(methoxy)pyridine-2-carboxamide, based on the total weight of the composition.

## Description

### TECHNICAL FIELD

The present invention relates to a transdermal absorption composition. More particularly, the present invention relates to a transdermal absorption composition comprising 4-cyclopropylmethoxy-N-(3,5-dichloro-1-oxido-4-pyridin-4-yl)-5-(methoxy)pyridine-2- carboxamide as an active ingredient.

### BACKGROUND ART

Cognitive impairment is a symptom observed in patients with Parkinson's disease (PD), and some patients may progress to dementia. Therefore, there is a need for drugs capable of providing prophylactic treatment to such patients. For example, animal studies have shown that phosphodiesterase 4 (PDE4) inhibitors such as Rolipram and Tilivapram are useful as drugs capable of providing prophylactic treatment. However, clinical studies have shown that PDE4 inhibitors are difficult to use in the treatment by oral administration because they induce emetogenic effects. Therefore, there is need to study routes of administration other than oral administration. Among such routes, transdermal administration is considered promising as an administration method that can maintain an effective plasma concentration at a constant level and does not cause side effects associated with oral administration.

As a technique for improving the transdermal absorbability of basic drugs, the use of fatty acid-based ionic liquids, which are equimolar salts of fatty acids and organic amine compounds, is known (Patent Document 1). However, depending on the type of drug, higher skin permeability may be required, and therefore, in some cases, it has been difficult to manufacture long-term stable formulations.

Tilivapram, which is a PDE4 inhibitor, is a compound having the chemical name 4-cyclopropylmethoxy-N-(3,5-dichloro-1-oxido-4-pyridin-4-yl)-5-(methoxy)pyridine-2-carboxamide, and has a chemical structure represented by the following formula: 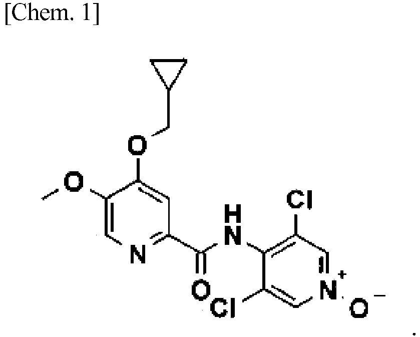 However, Tilivapram has low solubility in organic solvents and has therefore been considered to be difficult to use as transdermal administration formulations (transdermal absorption formulations).

In addition, in transdermal administration, a drug is mainly allowed to permeate into the epidermis, then diffuse into the dermis, and is absorbed through the capillaries of the subcutaneous tissue. Therefore, the penetration rate of the drug in a transdermal absorption-type formulation generally increases linearly at a certain time after administration, but decreases after a certain time has passed. The greater the amount of the drug contained in the formulation, the longer it takes for the permeation rate to decrease. Therefore, in order to prolong the duration of drug effect, it has been common to increase the amount of drug in the formulations.

### PRIOR ART DOCUMENTS

### PATENT DOCUMENTS

Patent Document 1: WO 2009/066457

### SUMMARY OF THE INVENTION

### TECHNICAL PROBLEM

An object of the present invention is to provide a transdermal absorption composition comprising 4-cyclopropylmethoxy-N-(3,5-dichloro-1-oxido-4-pyridin-4-yl)-5-(methoxy)pyridine-2-carboxamide (hereinafter also referred to as "Tilivapram").

### MEANS FOR SOLVING THE PROBLEM

In the development of a transdermal absorption composition comprising Tilivapram, the present inventors have intensively studied on various acidic compounds and consequently have found that levulinic acid is useful, and that Tilivapram can be efficiently utilized by adjusting the weight ratio of levulinic acid/Tilivapram to a specific range. Based upon the new findings, the present invention has been completed. The present inventors have also found that a phenomenon occurs in which the permeation rate can be maintained continuously when the content of Tilivapram in the formulation is kept below a certain amount. The present inventors have further found that the solubility of Tilivapram can be improved by further adding aromatic organic solvents, especially aromatic alcohols.

In other words, the present invention provides the following aspects.
[Item 1] A transdermal absorption composition comprising 4-cyclopropylmethoxy-N-(3,5-dichloro-1-oxido-4-pyridin-4-yl)-5-(methoxy)pyridine-2-carboxamide and levulinic acid, wherein the weight percentage of levulinic acid is 2 to 6 times the weight percentage of 4-cyclopropylmethoxy-N-(3,5-dichloro-1-oxido-4-pyridin-4-yl)-5-(methoxy)pyridine-2-carboxamide, based on the total weight of the composition.
[Item 2] The transdermal absorption composition according to Item 1, which further comprises an aromatic organic solvent.
[Item 3] The transdermal absorption composition according to Item 2, wherein the aromatic organic solvent is an aromatic alcohol.
[Item 4] The transdermal absorption composition according to Item 3, wherein the aromatic alcohol is benzyl alcohol.
[Item 5] The transdermal absorption composition according to any one of Items 1 to 4, wherein the content of 4-cyclopropylmethoxy-N-(3,5-dichloro-1-oxido-4-pyridin-4-yl)-5-(methoxy)pyridine-2-carboxamide is 5% by weight or less of the total composition.
[Item 6] The transdermal absorption composition according to any one of Items 1 to 5, wherein the content of 4-cyclopropylmethoxy-N-(3,5-dichloro-1-oxido-4-pyridin-4-yl)-5-(methoxy)pyridine-2-carboxamide is 2 to 5% by weight of the total composition.
[Item 7] The transdermal absorption composition according to any one of Items 1 to 6, wherein the transdermal absorption composition is an external preparation.
[Item 8] The transdermal absorption composition according to any one of Items 1 to 6, wherein the transdermal absorption composition is a patch preparation.
[Item 9] A patch preparation comprising a plaster laminated on one side of a support, wherein the plaster comprises the transdermal absorption composition according to any one of Items 1 to 6, a styrene-isoprene-styrene block copolymer, and a terpene resin.
   Furthermore, the present invention also provides the following aspects.
[Item 10] A method of treating cognitive impairment, which comprises administering a therapeutically effective amount of the transdermal absorption composition according to any one of Items 1 to 8 in a patient need thereof.
[Item 11] The transdermal absorption composition according to any one of Items 1 to 8 for use in the treatment of cognitive impairment.

### EFFECTS OF THE INVENTION

The present invention allows for good transdermal absorption of Tilivapram. The transdermal absorption composition of the present invention is expected to be used as a transdermal absorption formulation for the treatment of cognitive impairment.

### BRIEF DESCRIPTION OF THE DRAWINGS

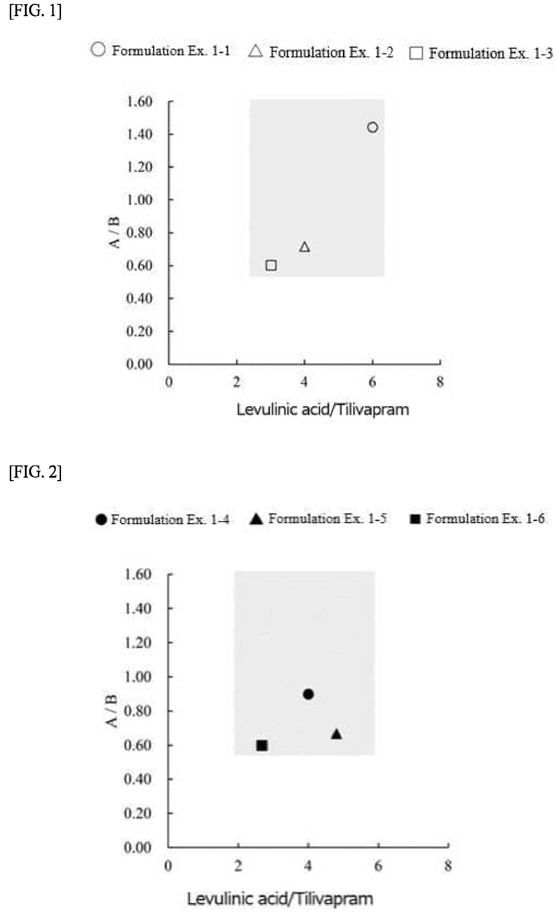

[FIG. 1] FIG. 1 shows the rate of change in drug skin permeation rate (A/B) of Formulation Examples 1-1 to 1-3 in the in vitro skin permeation test. "A" represents the drug skin permeation rate per hour (µg/cm²/hr) assumed from the cumulative skin permeation rate from 9 to 24 hours, and "B" represents the drug skin permeation rate per hour (µg/cm /hr) assumed from the cumulative skin permeation rate from 3 to 9 hours. The horizontal axis represents the weight ratio of levulinic acid/Tilivapram.
[FIG. 2] FIG. 2 shows the rate of change in drug skin permeation rate (A/B) of Formulation Examples 1-4 to 1-6 in the in vitro skin permeation test. "A" represents the drug skin permeation rate per hour (µg/cm²/hr) assumed from the cumulative skin permeation rate from 9 to 24 hours, and "B" represents the drug skin permeation rate per hour (µg/cm²/hr) assumed from the cumulative skin permeation rate from 3 to 9 hours. The horizontal axis represents the weight ratio of levulinic acid/Tilivapram.

### DETAILED DESCRIPTION OF THE PREFERRED EMBODIMENTS

The present invention is a transdermal absorption composition comprising 4-cyclopropylmethoxy-N-(3,5-dichloro-1-oxido-4-pyridin-4-yl)-5-(methoxy)pyridine-2-carboxamide and levulinic acid, wherein the content of levulinic acid is 2 to 6 times the weight of 4-cyclopropylmethoxy-N-(3,5-dichloro-1-oxido-4-pyridin-4-yl)-5-(methoxy)pyridine-2-carboxamide, based on the total weight of the composition.

In the transdermal absorption composition of the present invention, the content of Tilivapram may be any amount that has a therapeutic effect on the human body, e.g., 1% to 10%, preferably 2% to 5%, and more preferably 2% to 4%.

In the transdermal absorption composition of the present invention, the content of levulinic acid can be adjusted according to the content of Tilivapram. The content is, for example, 2 to 6 times the weight of Tilivapram, preferably 3 to 5 times the weight of Tilivapram.

The transdermal absorption composition of the present invention may comprise aromatic organic solvents. As used herein, the term "aromatic organic solvent" refers to an aromatic alcohol having hydroxyl group on the benzene ring and an aromatic ester having ester group on the benzene ring. Aromatic alcohols are preferred as aromatic organic solvents of the present invention.

Examples of the aromatic alcohols are benzyl alcohol, cinnamic alcohol, phenethyl alcohol, p-methylbenzyl alcohol, phenoxyethanol, 2-benzyloxyethanol, dimethylbenzylcarbinol, phenylethyl dimethyl carbinol, phenyl hexanol, glycol salicylate, and the like.

Examples of the aromatic esters include methyl benzoate, ethyl benzoate, benzyl benzoate, methyl salicylate, ethyl salicylate, and the like. Among them, benzyl alcohol and glycol salicylate are preferred.

The aromatic organic solvents are solvents capable of dissolving Tilivapram, and are preferably used in a weight of 2.5 times or more relative to the weight of Tilivapram.

The transdermal absorption composition of the present invention can be used in the form of external preparation. Examples of the external preparation include patches, ointments, creams, gels, gel creams, liniments, lotions, sprays, and aerosol formulations. These external formulations can be produced by methods commonly used in the pharmaceutical field.

### (Patch preparation)

When the transdermal absorption composition of the present invention is formulated as a patch, the patch is in the form of a non-aqueous patch preparation (tape preparation) that does not contain water as an essential ingredient. The patch consists of a support, a plaster, and a release liner. The plaster is a solid or semi-solid drug-containing layer at room temperature and is laminated on one side of the support.

### (Support)

The support can be used as long as it can hold the plaster and is commonly used for patch preparation. Examples of the support include woven fabrics, non-woven fabrics, and resin films. The support may be a laminate of woven fabric and film, a laminate of non-woven fabric and film, or a non-woven laminate film, for example, a polyester film or PET film laminated with a non-woven fabric.

Examples of the material for the support include polyester (e.g., polyethylene terephthalate (PET), polyethylene isophthalate, polypropylene terephthalate, polypropylene isophthalate, polybutylene terephthalate, or polyethylene naphthalate), polyolefin (polymer or copolymer of vinyl monomers such as ethylene, propylene, vinyl acetate, or acrylonitrile), polyamide (e.g., nylon or silk), polyurethane (PU), cellulose (e.g., cotton or hemp), and the like.

### (Plaster)

In the plaster of the present invention, a commonly-used base, for example, a base comprising additives such as an adhesive, a tackifier, and a softening agent can be used.

### (Adhesive)

Examples of the adhesive include rubber-based polymers, acrylic polymers, and silicone-based polymers.

Examples of the rubber-based polymer include synthetic rubbers such as styrene-isoprene-styrene block copolymer (hereinafter also referred to as "SIS"), styrene-butadienestyrene block copolymer, styrene-ethylene-butadiene rubber-styrene block copolymer, styrene-butadiene rubber, polyisoprene, polyisobutylene, polybutene, butyl rubber, silicone rubber; and natural rubbers. The rubber-based polymers are preferably SIS.

Examples of the acrylic polymer include polyacrylic acid methyl ester and polymethacrylic acid methyl ester. Specific examples thereof include DURO-TAK 87-2097, 87-2194, 87-2196, 87-2287, 87-2516, 87-2852, 87-235A (trade name, manufactured by Henkel Japan), Nisetsu KP-77, AS-370 (trade name, manufactured by Nippon Carbide Industry Co.).

Examples of the silicone-based polymer include dimethylpolysiloxane and diphenylpolysiloxane.

In the present invention, the content of the adhesive can be adjusted according to various factors such as the type of adhesive. The adhesive may be used alone or two or more thereof may be used in combination.

### (Tackifier)

The tackifier is a reagent mixed with rubber-based polymers such as SIS to improve the adhesion of the patch to the skin. Examples of the tackifier include polyterpene resin, polyolefin resin (e.g., plastibase), polystyrene resin, aromatic petroleum resin, rosin, hydrogenated rosin, and the like. The tackifier is preferably polyterpene resin and polyolefin resin (e.g., plastibase).

In the present invention, the content of the tackifier can be adjusted according to various factors such as the type of tackifier. The tackifier may be used alone or two or more thereof may be used in combination.

### (Softening agent)

The softening agent is a reagent mixed with rubber-based polymers such as SIS to give the polymers flexibility. Examples of the softening agent include petroleum-based softening agent such as polybutene, polyisobutylene, and process oil, fatty oil-based softening agent such as coconut oil and castor oil, purified lanolin, and liquid paraffin. The softening agent is preferably polybutene and liquid paraffin.

In the present invention, the content of the softening agent can be adjusted according to various factors such as the type of softening agent. The softening agent may be used alone or two or more thereof may be used in combination.

### (Additive)

The plaster may further comprise other additives to the extent that they do not interfere with the effects of the present invention. The additives include antifeedants, fillers, antioxidants, pH adjusters, thickeners, UV absorbers, surfactants, and others. These additives may be used alone or in combination of two or more thereof in appropriate amounts.

The antifeedant includes strong bitter substances such as denatonium, but is not limited thereto. It is preferably denatonium benzoate.

The content of the antifeedant is, for example, 0.01 to 0.1% of the total weight of the plaster. When denatonium benzoate is included as an antifeedant, the content thereof is preferably about 0.01 to about 0.1% of the total weight of the plaster. This amount is effective in preventing accidental ingestion by children, etc., due to the strong bitterness felt when the plaster is placed in the mouth.

The filler is not particularly limited as long as it is a solid powdered reagent used in plasters. Examples thereof include silicic anhydride, crystalline cellulose, zinc oxide, titanium dioxide, kaolin, calcium carbonate, and the like. In addition, it may be starch powders such as flour and cornstarch, carmellose, carmellose metal salt, carrageenan, carrageenan, pectin, powdered sugar, polyethylene powder, polystyrene sulfonate, and the like. Among them, crystalline cellulose, silicic anhydride, starch, carmellose, and carmellose metal salts are preferred.

The filler can be combined with several fillers of different bulk densities to adjust the voids formed accordingly. As the amount of the filler added increases, the adhesive strength of the patch improves, but if the amount is too large, the patch preparation becomes harder and the adhesive strength decreases.

Examples of the antioxidant include organic antioxidants such as dibutylhydroxyltoluene (BHT), propyl gallate and sodium ascorbate, and inorganic antioxidants such as sodium thiosulfate, sodium hydrogen sulfite, sodium sulfite and sodium pyrosulfite. The content of the antioxidant in the patch of the present invention can be adjusted according to various factors such as the type of antioxidants, but is, for example, 0.01 to 10% by weight of the total weight of the plaster. The antioxidant may be used alone or two or more thereof may be used in combination.

The pH adjuster may be any acid or base or their salts normally used for pH adjustment in the pharmaceutical field. Examples thereof include hydrochloric acid, sulfuric acid, phosphoric acid, citric acid, gluconic acid, succinic acid, acetic acid, methanesulfonic acid, edetoic acid, ammonia water, monoethanolamine, diethanolamine, triethanolamine, diisopropanolamine, triisopropanolamine, meglumine, tromethamol, glycine, potassium hydroxide, calcium hydroxide, sodium hydroxide, magnesium hydroxide, sodium citrate, sodium acetate, sodium bicarbonate potassium bicarbonate, and sodium bicarbonate. The content of the pH adjuster in the patch of the present invention can be adjusted according to various factors such as the type of pH adjuster, but is for example, 0.01 to 10% by weight of the total weight of the plaster. The pH adjuster may be used alone or two or more thereof may be used in combination.

The thickener includes Carbopol and the like, but is not limited thereto. The content of the thickener in the patch of the present invention can be adjusted according to various factors such as the type of thickener, but is, for example, 0.01 to 10% by weight of the total weight of the plaster. The thickener may be used alone or two or more thereof may be used in combination.

The UV absorber includes 2-hydroxy-4-methoxybenzophenone, t-butylmethoxydibenzoylmethane, para-aminobenzoic acid, and the like, but is not limited thereto. The content of the UV absorber in the patch of the present invention can be adjusted according to various factors such as the type of UV absorber, but is, for example, 0.01 to 10% by weight of the total weight of the plaster. The UV absorber may be used alone or two or more thereof may be used in combination.

The surfactant includes nonionic surfactants such as sorbitan monolaurate, anionic surfactants such as sodium lauryl sulfate, cationic surfactants such as benzalkonium chloride, and amphoteric surfactants such as betaine uryldimethylaminoacetic acid, but is not limited thereto. The content of the surfactant in the patch of the present invention can be adjusted according to various factors such as the type of surfactant, but is, for example, 0.01 to 10% by weight of the total weight of the plaster. The surfactant may be used alone or two or more thereof may be used in combination.

### (Release liner)

The release liner can be applied over the opposite side of the plaster with support to protect the plaster until the patch is applied to the skin. Examples of the release liner include films or sheets made of polyolefin such as polyethylene and polypropylene; ethylene-vinyl acetate copolymer, vinyl acetate-vinyl chloride copolymer, polyvinyl chloride, and the like; polyamide such as nylon; polyethylene terephthalate Polyester; cellulose derivatives; synthetic resins such as polyurethane, aluminum, paper, and other materials, and laminated materials thereof.

### (Method of manufacturing patch preparation)

In the present invention, the method of manufacturing a patch preparation comprises the step of mixing a plaster material comprising materials such as rubber-based polymer, terpene resin with a solvent to produce a coating solution, coating a release film or a support with the coating solution, and then drying it.

For example, a patch can be manufactured by mixing a plaster material comprising rubber-based polymer, terpene resin with a solvent to produce a coating solution, coating a release liner such as silicone-treated PET film with the coating solution, removing the solvent by heating and drying to obtain a plaster, and laminating the obtained plaster to a support such as woven fabric, nonwoven fabric, nonwoven fabric-laminated PET film. In addition, a patch can be manufactured by coating a support with the coating solution, removing the solvent by heating and drying to obtain a plaster, and laminating the obtained plaster to a release liner.

The resulting patch may be packaged in an appropriate package. The package includes a film bag, and the film is preferably a multilayer film, and more preferably an aluminum laminated film. The innermost layer is preferably made of polyethylene, polypropylene, or PET.

### (Ointments)

The ointment comprises a drug and further comprises, for example, higher fatty acids such as myristic acid or their esters, waxes such as whale wax, surfactants such as polyoxyethylene, and hydrocarbons such as hydrophilic vaseline.

### (Creams)

The cream comprises a drug and further comprises, for example, higher fatty acid esters such as myristate esters, water, hydrocarbons such as liquid paraffin, emulsifiers such as polyoxyethylene alkyl ethers.

### (Gel creams)

The gel cream is a formulation having properties intermediate between those of a gel and a cream, anis obtained by blending a gelling agent such as carboxy vinyl polymer and a neutralizing agent such as diisoprolanolamine with each ingredient of the above cream, and adjusting the pH to 4 to 8, or 5 to 6.5.

### (Liniments)

The liniment can be produced by blending a drug with various ingredients, for example, monovalent alcohols such as ethanol, propanol, and isopropyl alcohol or polyvalent alcohols such as polyethylene glycol, propylene glycol, and butylene glycol in an amount of 10 to 70%, water in an amount of 55 parts by mass or less, fatty acid esters in an amount of 60 parts by mass or less, and a surfactant (polyoxyethylene alkyl ether) in an amount of 10 parts by mass or less.

### (Lotions)

The lotion comprises a drug and further comprises lower alcohols such as ethanol, water and/or glycols. The lotion can be produced by mixing and stirring a drug, a skin irritation inhibitor, a lower alcohol, water and/or a glycol in appropriate amounts.

### (Sprays and aerosol formulations)

The spray and aerosol formulations can be produced by incorporating a drug and a skin irritation inhibitor in an amount of 0.1 to 30% by mass, based on the total amount of the formulation, into a known dosage form.

### EXAMPLES

The present invention is described in more detail below by way of examples, but the present invention is not limited in any way by these examples.

### Example 1: Evaluation of skin permeability of Tilivapram (1)

As described below, a patch comprising Tilivapram was prepared, and the skin permeability of Tilivapram was evaluated by an in vitro skin permeability test.

### (1) Production of patch comprising Tilivapram

Tilivapram, levulinic acid and benzyl alcohol were mixed and stirred to obtain uniform Solution A. Terpene resin dissolved in heptane and ethyl acetate, styrene-isoprene-styrene block copolymer (SIS5002) dissolved in heptane and ethyl acetate, oleyl alcohol, and fluid paraffin were mixed and stirred to obtain uniform Solution B.

Solution A was added to Solution B and mixed, followed by dissolving silicic anhydride and mixing uniformly to obtain an adhesive composition comprising Tilivapram. The resulting adhesive composition comprising Tilivapram was coated onto a release-treated PET film. Heptane and ethyl acetate were then removed by heating and drying to produce a patch comprising Tilivapram.

Formulation Example 1-1 (2%), Formulation Example 1-2 (3%), and Formulation Example 1-3 (4%) were prepared by varying the weight percentage of Tilivapram, respectively. The composition (% by weight) of each ingredient is shown in Table 1 below.

### (2) In vitro skin permeability test

According to in vitro skin permeability tests using Franz cells, the skin permeability of the drug was evaluated for the patches of Formulation Examples 1-1 to 1-3. The skin used for the test was the back excised skin of a miniature pig (Yucatan micropig, 4 months old). The cumulative skin permeation (µg/cm²) at 3, 6, 9, and 24 hours after the start of the test is shown in Table 1.

The drug skin permeation rate per hour (µg/cm²/hr) assumed from the cumulative skin permeation from 9 to 24 hours (A) and the drug skin permeation rate per hour (µg/cm²/hr) assumed from the cumulative skin permeation from 3 to 9 hours (B) were calculated, and the rate of change in drug skin permeation rate (A/B) was calculated. The results are shown in Table 1.

**[Table 1]**

| | | **Formulation Ex. 1-1** | **Formulation Ex. 1-2** | **Formulation Ex. 1-3** |
|---|---|---|---|---|
| Tilivapram | | 2 | 3 | 4 |
| Levulinic acid | | 12 | 12 | 12 |
| Benzyl alcohol | | 16 | 16 | 16 |
| Oleyl alcohol | | 7 | 7 | 7 |
| Silicic anhydride | | 5 | 5 | 5 |
| Liquid paraffin | | 7.5 | 7.5 | 7.5 |
| Terpene resin | | 40.5 | 39.5 | 38.5 |
| SIS5002 | | 10 | 10 | 10 |
| Total (% by weight) | | 100.0 | 100.0 | 100.0 |
| Levulinic acid/Tillivapram | | 6 | 4 | 3 |
| Cumulative skin penetration (µg/cm²) | 3 hr | 0.8 | 1.8 | 1.4 |
| | 6 hr | 4.9 | 13.5 | 10.4 |
| | 9 hr | 11.1 | 29.2 | 22.3 |
| | 24 hr | 48.3 | 78.1 | 53.6 |
| Drug skin permeation rate per hour assumed from cumulative skin permeation from 9-24 hr (A) | | 2.48 | 3.26 | 2.09 |
| Drug skin permeation rate per hour assumed from cumulative skin permeation from 3-9 hr (B) | | 1.72 | 4.55 | 3.47 |
| A/B | | 1.4 | 0.7 | 0.6 |

The formulation of Formulation Example 1-2 had the highest cumulative skin permeation after 24 hours, indicating that it had the highest skin permeability. In conventional patches, the cumulative skin permeation tends to be higher as the amount of drug increases. On the other hand, in the patches of the present invention, it was observed that the skin permeation of the drug decreased as the amount of drug increased.

The rate of change in drug skin permeation rate (A/B) in the formulations of Formulation Examples 1-1 to 1-3 is shown in FIG. 1. The horizontal axis represents the weight ratio of levulinic acid/Tilivapram.

When a transdermal absorption formulation is applied to the skin, the blood concentration of the drug increases in proportion to the drug skin penetration rate (Flux). Therefore, a small change in Flux leads to a small change in blood concentration, and it is considered ideal for Flux to remain almost unchanged. If the change in blood concentration is small, it is easy to maintain the blood concentration above the blood concentration at which the drug is effective and below the blood concentration at which side effects occur. A Large change in blood concentration is undesirable because the change can induce drug effects but also side effects, or reduce the dose to reduce side effects and shorten the time of drug effect. The transdermal formulation of Tilivapram should be able to maintain its absorption rate at least longer than the oral formulation to reduce side effects. Since the absorption of oral formulation converges after a few hours, it is necessary to maintain absorption for longer. Furthermore, it is more desirable for the blood concentration of tilivapram to reach a steady state.

In the bioequivalence studies of pharmaceutical products, the allowable range of change in blood concentration is defined as 80% to 125%. The minimum and maximum values of the rate of change (A/B) in Flux from 3 to 9 hours and from 9 to 24 hours, respectively, from the allowable range of change in blood concentration are 0.6 (80/125) to 1.6 (125/80). The desired therapeutic effect with reduced side effects can be expected if the rate of change in Flux is within this range.

As shown in Table 1 and FIG. 1, Formulation Example 1-1 (1.4), Formulation Example 1-2 (0.7) and Formulation Example 1-3 (0.6) were within the above range (0.6 to 1.6). The results showed that when the weight ratio of levulinic acid/Tilivapram is 3 to 6, the change in blood concentration is smaller than that of oral administration, and the blood concentration of Tilivapram is more likely to reach a steady state, making these formulations even less likely to cause side effects.

### Example 2: Evaluation of skin permeability of Tilivapram (2)

The skin permeability of Tilivapram was evaluated according to the same method as in Example 1. In this test, Formulation Examples 1-4 to 1-6 in Table 2 below, in which the amount of levulinic acid is adjusted, were used.

The cumulative skin permeation (µg/cm²) at 3, 6, 9, and 24 hours after the start of the test, the drug skin permeation rate per hour (µg/cm²/hr) assumed from the cumulative skin permeation from 9 to 24 hours (A) and the drug skin permeation rate per hour (µg/cm²/hr) assumed by the cumulative skin permeation from 3 to 9 hours (B), and the rate of change in drug skin permeation rate (A/B) of Formulation Examples 1-4 to 1-6 are shown in Table 2. In addition, the rate of change in drug skin permeation rate (A/B) of the formulations of Formulation Examples 1-4 to 1-6 are shown in FIG. 2. The horizontal axis represents the weight ratio of levulinic acid/Tilivapram.

**[Table 2]**

| | | **Formulation Ex.1-4** | **Formulation Ex.1-5** | **Formulation Ex.1-6** |
|---|---|---|---|---|
| Tilivapram | | 2.5 | 2.5 | 3 |
| Levulinic acid | | 10 | 12 | 8 |
| Benzyl alcohol | | 16 | 16 | 16 |
| Oleyl alcohol | | 7 | 7 | 7 |
| Silicic anhydride | | 5 | 5 | 5 |
| Liquid paraffin | | 7.5 | 10 | 14 |
| Terpene resin | | 42 | 41.5 | 38 |
| SIS5002 | | 8 | 8 | 9 |
| Total (% by weight) | | 100.0 | 100.0 | 100.0 |
| Levulinic acid/Tilivapram | | 4 | 4.8 | 2.67 |
| Cumulative skin penetration (µg/cm²) | 3 hr | 2.4 | 2.9 | 2.2 |
| | 6 hr | 11.3 | 15.5 | 8.5 |
| | 9 hr | 23.4 | 32.8 | 17.8 |
| | 24 hr | 79.3 | 89.6 | 44.3 |
| Drug skin permeation rate per hour assumed from cumulative skin permeation from 9-24 hr (A) | | 3.73 | 3.79 | 1.77 |
| Drug skin permeation rate per hour assumed from cumulative skin permeation from 3-9hr (B) | | 4.02 | 5.76 | 3.09 |
| A/B | | 0.9 | 0.7 | 0.6 |

As shown in Table 2 and FIG. 2, Formulation Example 1-4 (0.9), Formulation Example 1-5 (0.7) and Formulation Example 1-6 (0.6) were within the above range (0.6 to 1.6). The results showed that even if the weight ratio of levulinic acid/Tilivapram is less than 3, the change in blood concentration is smaller than that of oral administration, and the blood concentration of Tilivapram can easily reach a steady state, and thus these formulations can be used with fewer side effects.

### INDUSTRIAL APPLICABILITY

The present invention allows for good transdermal absorption of Tilivapram. The transdermal absorption composition of the present invention is expected to be used as a transdermal absorption formulation for the treatment of cognitive impairment.

## Claims

1. A transdermal absorption composition comprising 4-cyclopropylmethoxy-N-(3,5-dichloro-1-oxido-4-pyridin-4-yl)-5-(methoxy)pyridine-2-carboxamide and levulinic acid, wherein the weight percentage of levulinic acid is 2 to 6 times the weight percentage of 4-cyclopropylmethoxy-N-(3,5-dichloro-1-oxido-4-pyridin-4-yl)-5-(methoxy)pyridine-2-carboxamide, based on the total weight of the composition.

2. The transdermal absorption composition according to claim 1, which further comprises an aromatic organic solvent.

3. The transdermal absorption composition according to claim 2, wherein the aromatic organic solvent is an aromatic alcohol.

4. The transdermal absorption composition according to claim 3, wherein the aromatic alcohol is benzyl alcohol.

5. The transdermal absorption composition according to claim 1, wherein the content of 4-cyclopropylmethoxy-N-(3,5-dichloro-1-oxido-4-pyridin-4-yl)-5-(methoxy)pyridine-2-carboxamide is 5% by weight or less of the total composition.

6. The transdermal absorption composition according to claim 1, wherein the content of 4-cyclopropylmethoxy-N-(3,5-dichloro-1-oxido-4-pyridin-4-yl)-5-(methoxy)pyridine-2-carboxamide is 2 to 5% by weight of the total composition.

7. The transdermal absorption composition according to claim 1, wherein the transdermal absorption composition is an external preparation.

8. The transdermal absorption composition according to claim 1, wherein the transdermal absorption composition is a patch preparation.

9. A patch preparation comprising a plaster laminated on one side of a support, wherein the plaster comprises the transdermal absorption composition according to any one of claims 1 to 6, a styrene-isoprene-styrene block copolymer, and a terpene resin.
